Europäisches Patentamt

European Patent Office

Office européen des brevets

(11) Publication number: **0 095 180**
**B1**

## (12) EUROPEAN PATENT SPECIFICATION

(45) Date of publication of patent specification: **15.10.86**

(21) Application number: **83105103.2**

(22) Date of filing: **24.05.83**

(51) Int. Cl.⁴: **A 61 F 5/48, G 01 N 27/04**

(54) Alarm device for use in a baby's diaper.

(30) Priority: **21.05.82 KR 823982**
**22.07.82 US 400816**

(43) Date of publication of application:
**30.11.83 Bulletin 83/48**

(45) Publication of the grant of the patent:
**15.10.86 Bulletin 86/42**

(84) Designated Contracting States:
**BE CH DE FR GB IT LI NL**

(56) References cited:
**DE-A-2 727 930**
**DE-U-7 102 612**
**GB-A-1 192 421**
**US-A-4 205 672**

(73) Proprietor: **Nae Wae Electric Co., Ltd.**
**70-27, Yekwan-dong**
**Joong-ku Seoul (KR)**

(72) Inventor: **Yoo, Kil-Soo**
**1066 Juahn 3 dong**
**Nam-ku Inchon (KR)**

(74) Representative: **Lehn, Werner, Dipl.-Ing. et al**
**Hoffmann, Eitle & Partner Patentanwälte**
**Arabellastrasse 4 (Sternhaus)**
**D-8000 München 81 (DE)**

Courier Press, Leamington Spa, England.

**Description**

The present invention relates to an alarm device for use in a baby's diaper and, more particularly, to a diaper alarm in which a buzzer sounds after a fecal excretion is detected.

Up to this time, a baby's excrement has usually been detected only by opening the baby's diaper, so that there were disadvantages such as conditions unsanitary for the baby and the potential for skin diseases.

It is true that various electrical moisture alarms have been proposed, but these have disadvantages as will be discussed in the following.

DE—A—27 27 930 discloses a self-adhesive moisture alarm comprising a small housing in which is contained an electrical circuit including a self-blocking oscillator, a transistor and a battery. Two sensor electrodes protrude from the housing and are connected respectively to the base and collector of the transistor.

No further precise details of the housing or form of the electrodes, or of the precise circuit are disclosed. Suggested uses of the device include, however, detection of bed-wetting, for which purpose the electrodes are to be embedded in damp-sensitive matting. To permit this, the electrodes are clearly elongate protruding members, quite unsuitable for use within a baby's diaper, and the document does not suggest such use.

US—A—4 205 672 discloses a conductivity sensor for diapers. However, this device requires use of special diapers containing an electrically conductive layer. A pair of jaws has sharp protrusions for penetrating the diaper and contacting the conductive layer. This proposal is not only expensive (requiring special diapers) but also uncomfortable and possibly dangerous for the baby.

DE—U—7 102 612 suggests a signalling apparatus for detection of moisture in a baby's diaper. However, the apparatus is to be positioned away from the baby and is connected to a pair of electrodes embedded in the diaper via a spiral connection cable. The impracticalities and dangers of such an arrangement are obvious. The baby could easily become entangled in the connection cable with unfortunate consequences.

From the above discussion it will be clear that there is a need for a simple, inexpensive, reliable and safe moisture alarm for use in a baby's diaper.

An object of the invention is to supply this need.

According to the invention, there is provided an alarm device for use in a baby's diaper comprising a casing formed in two parts with a space being formed between the two parts, a circuit board including an electronic circuit being provided in said space, a moisture detecting means comprising a first detecting part and a second detecting part, the first and second detecting parts being fixed in recesses formed on the outer surface of a first part of the casing and means for generating audible sound being secured inside the second part of the casing and over a hole formed in said second part, said detecting means, said electronic circuit and said sound generating means being electrically connected such that when, due to moisture outside the device, a path for the flow of electricity between the first and second detecting parts exists the sound generating means is actuated by the electronic circuit to generate audible sound.

Thus, the presence of the baby's excrement is indicated by the sounding of an alarm, e.g. a buzzer provided in the diaper in response to detection of moisture from the excrement which is absorbed by the diaper. Thus, the baby can be effectively protected from unsanitary conditions and skin diseases.

The alarm device of the present invention is preferably sufficiently small as to be placed at the outer surface of the diaper or at the inner surface of the baby's panties, if desired.

An embodiment of the present invention will be described by way of example with reference to the drawings, in which:

Figure 1 is an exploded perspective view of an alarm device for use in a baby's diaper and embodying the present invention;

Figure 2 is a fragmentary perspective view of the alarm device of Figure 1;

Figure 3 is a sectional side view of the alarm device of Figure 1;

Figure 4 is an enlarged view of part of Figure 3; and

Figure 5 is a schematic diagram of a circuit which is part of the device of Figure 1.

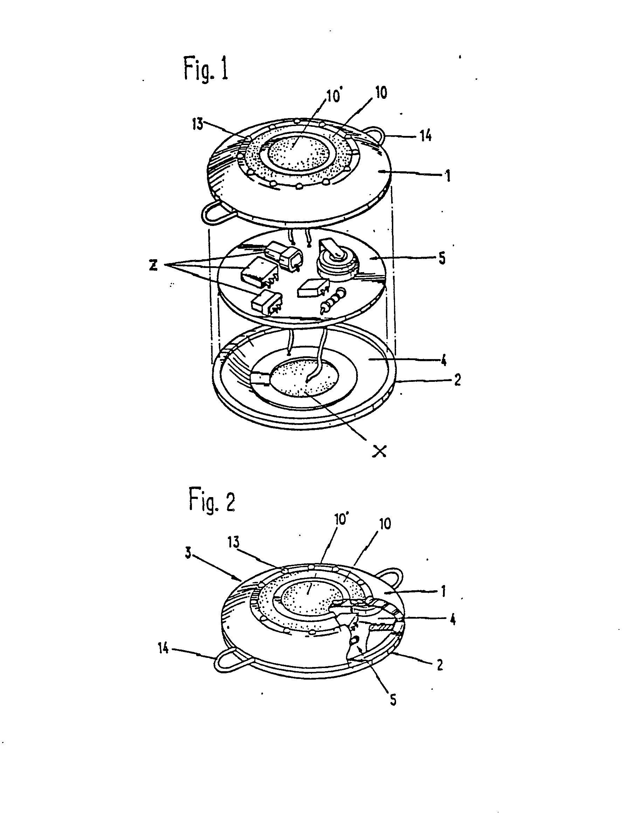

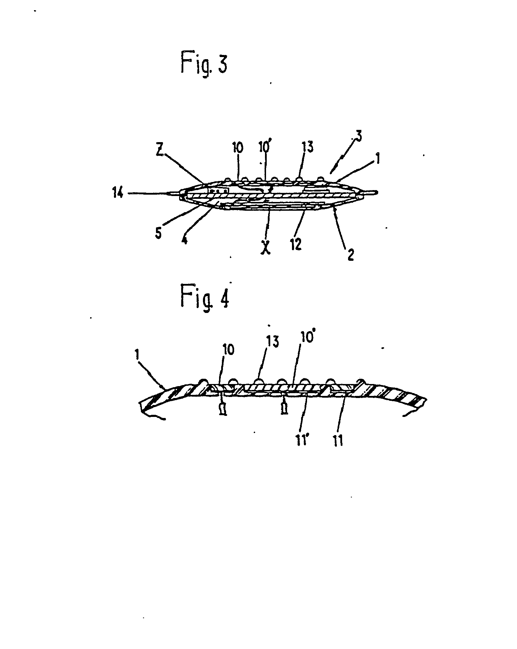

As shown in Figures 1, 2, 3 and 4, an alarm device 3 has a housing which includes a case 1 and cover 2 having a circular, square, polygon or other shape perimeter, here, circular. A space 4 is formed between the case 1 and cover 2, and an insulated circuit board 5 having an electronic circuit Z thereon is disposed in the space 4. A detecting part 10 of ring shape and a detecting part 10' of circular shape are concentrically set into recesses 11 and 11', respectively, and the ring shaped detecting part 10 and circular detecting part 10' are electrically connected with the electronic circuit Z, the recesses 11 and 11' being centrally formed in the case 1.

A buzzer X is secured to the inside of the cover 2 by securing the edges of the buzzer X to the cover inner surface around a round hole 12 formed in the center of the cover 2.

A number of small rounded projections 13 are formed on the case 1 along the outer edge of the detecting part 10 in order to prevent the case 1 from slipping. Two tie-rings 14 are furnished on opposite sides of the case 1.

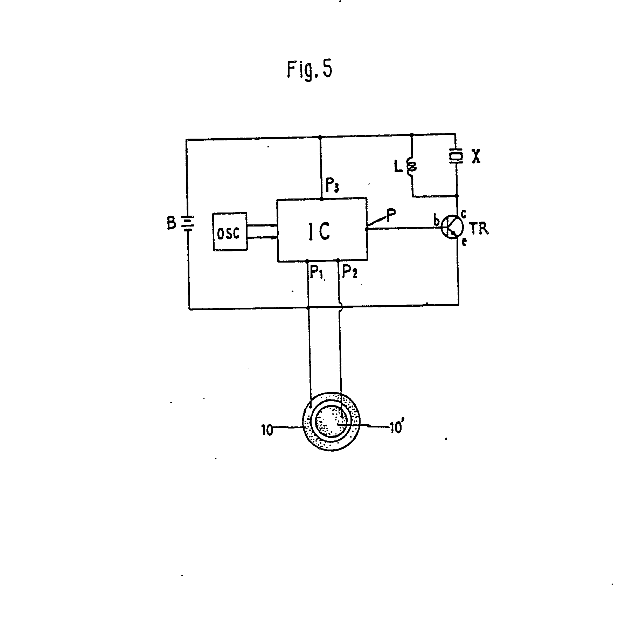

As shown in Figure 5, the circuit used in the alarm device 3 is designed so that electricity is supplied from the battery B to integrated circuit IC through the electric wires $P_1$ and $P_3$, integrated circuit IC is connected by wires $P_1$ and $P_2$ to the detecting parts 10 and 10', an oscillator OSC is connected directly to the integrated circuit IC, and the terminal P of the integrated circuit IC is connected with the base b of a transistor TR. The collector c of transistor TR is connected to one end of the buzzer X, the other end of the buzzer X is connected to the battery B, a coil L is connected in parallel with the buzzer X, and the emitter e of the transistor TR is directly connected to the negative power supply terminal of the battery B.

The detecting parts 10 and 10' are preferably made of a conductive metal, moisture which comes into contact therewith providing a path for the flow of electricity between the detecting parts 10 and 10'.

•In using the present invention, the alarm device 3 is positioned appropriately in a baby's diaper or panties so as to allow the detecting parts 10 and 10' to contact the diaper. In placing the alarm device 3 inside of panties, the tie-rings 14 are used for securing it to the panties with some appropriate fastener.

When the baby relieves itself, moisture is absorbed by the diaper and the detecting parts 10 and 10' of the alarm device 3 detect the moisture and cause integrated circuit IC to send a signal to the base b of the transistor TR which is amplified by the transistor TR and causes the buzzer X to oscillate and produce an audible alarm indicating excrement is present. At this time, the coil L resonates with the buzzer X to make the alarm sound louder by increasing the voltage of the oscillations.

The oscillator OSC supplies a frequency required by the integrated circuit IC.

The alarm device 3 of the present invention produces the alarm by means of the electronic circuit Z which is placed in the space 4 formed between the case 1 and cover 2 and made with small dimensions, and the detecting parts 10 and 10' are fixed in the recesses 11 and 11', respectively, to make the overall dimensions of the alarm device smaller.

As hereinbefore mentioned, the detecting parts 10 and 10' contact the diaper during use so that the parts 10 and 10' can detect any excrement immediately, and the buzzer X is secured inside the cover 2 and over a round hole 12 provided in the middle part of the cover 2. Thus, all the essential parts are put into the case 1 and cover 2, which are preferably made of flat plastic and of small size so that the alarm device is very handy to use and a baby can be protected from unsanitary conditions and skin diseases.

Although a particular preferred embodiment of the invention has been disclosed in detail for illustrative purposes, it will be recognized that variations or modifications of the disclosed apparatus, including the rearrangement of parts, lie within the scope of the present invention as defined in the appended claims.

## Claims

1. An alarm device for use in a baby's diaper comprising a casing formed in two parts (1, 2) with a space (4) being formed between the two parts, a circuit board (5) including an electronic circuit (z) being provided in said space (4), a moisture detecting means comprising a first detecting part (10) and a second detecting part (10'), the first and second detecting parts (10, 10') being fixed in recesses formed on the outer surface of a first part (1) of the casing and means (x) for generating audible sound being secured inside the second part (2) of the casing and over a hole formed in said second part (2), said detecting means (10, 10'), said electronic circuit (z) and said sound generating means (x) being electrically connected such that when, due to moisture outside the device, a path for the flow of electricity between the first and second detecting parts (10, 10') exists the sound generating means (x) is actuated by the electronic circuit (z) to generate audible sound.

2. The alarm device as claimed in claim 1, wherein an integrated circuit (IC), to which electricity may be supplied from a battery (B), is connected to said detecting parts (10, 10'), an oscillator (OSC) is connected directly with said integrated circuit, a terminal (P) of said integrated circuit is connected with the base of a transistor (TR), the collector of said transistor is connected to one end of said alarm means, the other end of said alarm means is connected to a terminal for connection to said battery, a coil (L) is connected in parallel with said alarm means, and the emitter of said transistor is connected to a terminal for connection to said battery (B).

3. An alarm device according to claim 1 or 2, further comprising a plurality of projections (13) provided on said case (1) in the region of said detecting parts, and including at least one tie-ring (14) provided on said case for securing said alarm device to the diaper.

4. An alarm device according to any one of claims 1 to 3 wherein said sound generating means comprises a buzzer (x).

5. An alarm device according to any one of the preceding claims wherein said first (10) and second (10') detecting parts are arranged mutually concentrically on said casing (1, 2).

6. An alarm device according to claim 5 wherein one of said detecting parts is annular.

7. An alarm device according to claim 6 wherein the other of said detecting parts is a circular member within said annular part.

## Revendications

1. Dispositif d'alarme à utiliser dans une couche de bébé, comprenant un boîtier constitué de deux parties (1, 2) avec un espace (4) qui est formé entre ces deux parties, une carte de circuit (5) contenant un circuit électronique (Z) étant logée dans le dit espace (4), un organe de détection d'humidité comprenant un premier élément détecteur (10) et un second élément détecteur (10'), le premier et le second éléments détecteurs (10, 10') étant fixés dans des évidements réalisés sur la face externe d'une première partie (1) du boîtier et un moyen (X) pour la production d'un son audible étant fixé à l'intérieur de la seconde partie (2) du boîtier et par-dessus un trou pratiqué dans cette seconde partie (2), le susdit organe détecteur (10, 10'), le susdit circuit électronique (Z) et le susdit moyen de production d'un son (X) étant électriquement connectés de façon que quand, en cas d'humidité en dehors du dispositif, il existe un trajet pour l'écoulement de l'électricité entre les premier et second éléments détecteurs (10, 10'), le moyen de production du son (X) est déclenché par le circuit électronique (Z) pour engendrer un signal sonore.

2. Dispositif d'alarme selon revendication 1, dans lequel un circuit intégré (IC), qui peut être alimenté électriquement par une pile (B), est connecté aux susdits éléments détecteurs (10, 10'), un oscillateur (OSC) est relié directement au dit circuit intégré, une borne (P) de ce circuit intégré est raccordée à la base d'un transistor (TR), le collecteur du dit transistor est connecté à une borne du dit moyen d'alarme, l'autre borne de ce moyen d'alarme est connectée à une borne pour le raccordement de la pile, une bobine (L) est branchée en parallèle avec le susdit moyen d'alarme et l'émetteur du susdit transistor est relié à une borne pour le raccordement à la susdite pile (B).

3. Dispositif d'alarme selon la revendication 1 ou 2, comprenant en outre un certain nombre de saillies (13) réalisées sur le capot (1) dans la zone des susdits éléments détecteurs, et possédant au moins un anneau d'attache (14) installé sur le dit capot pour fixer le dispositif d'alarme à la couche.

4. Dispositif d'alarme selon l'une quelconque des revendications 1 à 3, dans lequel de moyen de production du signal sonore comprend un vibreur (X).

5. Dispositif d'alarme selon l'une quelconque des revendications précédentes, dans lequel les premier (10) et second (10') éléments détecteurs sont disposés mutuellement de façon concentrique sur le susdit boîtier (1, 2).

6. Dispositif d'alarme selon la revendication 5, dans lequel l'un des susdits éléments détecteurs est annulaire.

7. Dispositif d'alarme selon la revendication 6, dans lequel l'autre des susdits éléments détecteurs est un élément circulaire à l'intérieur de l'élément annulaire.

## Patentansprüche

1. Alarmeinrichtung zur Verwendung in einer Babywindel, umfassend ein Gehäuse, welches in zwei Teilen (1, 2) ausgebildet ist mit einem Zwischenraum (4), der zwischen den beiden Teilen gebildet ist, eine gedruckte Schaltung (5), umfassend eine elektronische Schaltung (z), welche in dem Zwischenraum (4) vorgesehen ist, eine Feuchtigkeitsdetektor-Einrichtung mit einem ersten Detektorteil (10) und einem zweiten Detektorteil (10, 10'), wobei das erste und zweite Detektorteil (10, 10') in Ausnehmungen fixiert ist, welche an der äußeren Oberfläche eines ersten Teiles des Gehäuses ausgebildet sind, sowie eine Einrichtung (x) zur Erzeugung eines hörbaren Tones, welche innerhalb des zweiten Teils (2) des Gehäuses sowie über eine Ausnehmung, die in dem zweiten Teil (2) ausgebildet ist, gesichert ist, wobei die Detektoreinrichtung (10, 10'), die elektronische Schaltung (z) und die Tonerzeugungs-Einrichtung (x) elektrisch so angeschlossen sind, daß dann, wenn aufgrund von Feuchtigkeit außerhalb der Vorrichtung ein Pfad für den Elektrizitätsfluß zwischen dem ersten und zweiten Detektorteil (10, 10') existiert, die Tonerzeugungs-Einrichtung (x) durch die elektrische Schaltung (z) betätigt wird, um den hörbaren Ton zu erzeugen.

2. Alarmeinrichtung nach Anspruch ein, dadurch gekennzeichnet, daß eine integrierte Schaltung (IC), der Elektrizität von einer Batterie (B) zugeführt werden kann, mit den Detektorteilen (10, 10') verbunden ist, daß ein Oszillator (OSC) direkt mit der integrierten Schaltung verbunden ist, daß ein Anschluß (P) der integrierten Schaltung mit der Basis eines Transistors (TR) verbunden ist, dessen Kollektor mit einem Ende der Alarmeinrichtung verbunden ist, wobei das andere Ende der Alarmeinrichtung an einen Anschluß zum Verbinden mit der Batterie angeschlossen ist, daß eine Spule (L) parallel zur Alarmeinrichtung angeschlossen ist und daß der Emitter des Transistors mit dem Anschluß zur Verbindung mit der Batterie (B) verbunden ist.

3. Alarmeinrichtung nach Anspruch 1 oder 2, dadurch gekennzeichnet, daß sie außerdem eine Vielzahl von Vorsprüngen (13) umfaßt, die am Gehäuse (1) im Gebiet der Detektorteile vorgesehen sind und mindestens einen Befestigungsring (14) umfaßt, welcher am Gehäuse zur Sicherung bzw. Befestigung der Alarmvorrichtung an der Windel vorgesehen ist.

4. Alarmeinrichtung nach einem der Ansprüche 1 bis 3, dadurch gekennzeichnet, daß die Tonerzeugungseinrichtung einen Schnarrer (x) umfaßt.

5. Alarmvorrichtung nach einem der vorhergehenden Ansprüche, dadurch gekennzeichnet, daß das erste (10) und zweite (10') Detektorteil gegenseitig konzentrisch auf dem Gehäuse (1, 2) angeordnet sind.

6. Alarmeinrichtung nach Anspruch 5, dadurch gekennzeichnet, daß einer der Detektorteile ringförmig ist.

7. Alarmeinrichtung nach Anspruch 6, dadurch gekennzeichnet, daß das andere Detektorteil ein kreisförmiges Glied innerhalb des ringförmigen Teiles ist.

Fig. 1

Fig. 2

Fig. 3

Fig. 4

## Fig.5